# EUROPÄISCHE PATENTSCHRIFT

(11) **EP 1 029 862 B1**
(45) Veröffentlichungstag und Bekanntmachung des Hinweises auf die Patenterteilung: **20.06.2001**
(21) Anmeldenummer: 99103354.9
(22) Anmeldetag: 20.02.1999
(51) Int. Cl.: C07D 451/06

(54) **Verfahren zum Herstellen von endo-Nortropin unter Verwendung von 8-Benzyl-nortropan-3-on-perchlorat als Zwischenprodukt, sowie letzteres Salz selbst**
Process for the preparation of endo-nortropine using 8-benzyl-nortropan-3-one-perchlorate as intermediate, as well as said salt
Procédé pour la préparation d'endo-nortropine utilisant de 8-benzyl-nortropan-3-one-perchlorate comme produit intermédiaire, ainsi que ledit sel

(43) Veröffentlichungstag der Anmeldung: 23.08.2000
(73) Patentinhaber: Dr. R. Pfleger Chemische Fabrik GmbH, 96052 Bamberg (DE)
(72) Erfinder: Sachse, Rolf, 13465 Berlin (DE); Schaupp, Albert, 96129 Strullendorf (DE)
(74) Vertreter: Fleck, Thomas, Dr. Dipl.-Chem.

(56) Entgegenhaltungen:
- EP-A- 0 042 705
- DE-A- 3 546 218
- DE-B- 1 194 422
- CHEMICAL ABSTRACTS, vol. 58, no. 4, 18. Februar 1963 (1963-02-18) Columbus, Ohio, US; abstract no. 3384g, Spalte 3384; XP002109159 & K. NADOR ET AL.: ARZNEIMITTEL-FORSCHUNG, Bd. 12, 1962, Seiten 305-9,

## Beschreibung

Die Erfindung betrifft ein Verfahren zum Herstellen von endo-Nortropin unter Verwendung von 8-Benzyl-nortropan-3-on-perchlorat als Zwischenprodukt, sowie letzteres Salz selbst.

Im Stand der Technik werden verschiedene Verfahren zur Herstellung von endo-Nortropin und diese Substanz selbst beschrieben. Endo-Nortropin ist das Schlüsselprodukt zur Herstellung der wichtigen Azoniaspironortropanolester, die als Pharmazeutika, insbesondere als Spasmolytika, eingesetzt werden (vergl. insbesondere hierzu die DE-PS 1 194 422), während das 8-Benzyl-nortropan-3-on-perchlorat bisher unbekannt ist.

Bisher sind vom 8-Benzyl-nortropan-3-on nur das Picrat, das Hydrochlorid und das Hydrobromid bekannt, für die jedoch keine weiteren Anwendungsmöglichkeiten genannt werden. Das 8-Benzyl-nortropan-3-on wird auch in der EP-A1 0042 705 als Zwischenprodukt bei der Herstellung von Azabicycloalkanderivaten erwähnt, deren pharmakologische Aktivität geschildert wird.

Im Prinzip sind drei verschiedene Verfahrenswege zur Herstellung von endo-Nortropin bekannt, nämlich die Verseifung von Tropanalkaloiden, wie Nor-1-hyoscyamin, Convolvin und Convolamin. Die Verseifung der drei Alkaloide ist jedoch nicht rentabel, da die Ausgangsprodukte selten und teuer sind. Deshalb hat man bekanntermaßen Tropin oxydativ demethyliert, um endo-Nortropin herzustellen. Außerdem sind noch photochemische Methoden zur Demethylierung und die Demethylierung mit Chlorameisensäure-Ethylester bekannt geworden. So beispielsweise aus der DE-A1 35 46 218. Nachteilig hat sich bei diesem Verfahren der Dealkylierung bemerkbar gemacht, daß teure, zum Teil umweltschädliche, Chemikalien als Ausgangsprodukte eingesetzt werden müssen, die ihrerseits ebenfalls nur umständlich herzustellen sind. Auch arbeiten die bekannten Verfahren überwiegend mit Überdruck und sind meistens nicht stereoselektiv.

Der vorliegenden Erfindung liegt deshalb die Aufgabe zugrunde, ein alternatives Verfahren zum Herstellen von endo-Nortropin zu schaffen, welches einfach und schnell in wirtschaftlicher Weise durchgeführt werden kann, und das zudem umweltfreundlich ist. Ein weiteres Ziel ist es, möglichst auf etwaige Überdruckverfahrensstufen verzichten zu können und trotzdem möglichst stereoselektiv zu arbeiten. Letzendlich soll damit eine wirtschaftliche Herstellung von Trospiumchlorid ermöglicht werden.

In überraschender Weise wird diese Aufgabe durch das eingangs genannte Verfahren gelöst, welches auf einer zweistufigen Behandlung von 8-Benzyl-nortropan-3-on-perchlorat mit katalytisch erregtem Wasserstoff basiert, wobei man das Startprodukt zunächst in wässriger Suspension bei atmosphärischem Druck und Raumtemperatur mit einem Palladium-Katalysator vorhydriert, am Ende der Reaktion den Katalysator durch Filtration zurückgewinnt, das Filtrat über einen Anionenaustauscher schickt und die nunmehr alkalisch reagierende Lösung mit durch Raney-Nickel erregtem Wasserstoff bei atmosphärischem Druck und Raumtemperatur bei 1000 - 1500 Umdrehungen pro Minute turbuliert.

Von besonderer erfindungsgemäßer Bedeutung ist die Tatsache, daß als neuer Stoff 8-Benzyl-nortropan-3-on-perchlorat zunächst als Zwischenprodukt hergestellt wird, und zwar durch die an sich bekannte Robinson-Schöpf-Methode und anschließender Zugabe von Perchlorsäure und Ausfällen des entsprechenden Perchlorat-Salzes.

Anschließend wird das Perchlorat in wässriger Suspension bei Raumtemperatur von vorzugsweise 20°C unter Normaldruck mit katalytisch erregtem Wasserstoff behandelt. Als Katalysator fungiert dabei ein gebrauchsfertiger Palladium-Katalysator, 10prozentig, auf Aktivkohle.

Bei diesem Verfahren wird die Benzylgruppe abgespalten und das Substrat zum 3-Nortropanon umgesetzt, das dann als Salz der Perchlorsäure in Lösung geht.

Nach dem Abfiltrieren des Katalysators schickt man die wässrige Lösung über einen stark basischen Anionenaustauscher vom Typ Styrol-Divinylbenzol der das Gegenion OH⁻ aufweist.

Die nunmehr basische, wässrige Lösung von 3-Nortropanon wird auf Zusatz von Raney-Nickel-Katalysator mit Wasserstoff zum endo-Nortropin hydriert.

Die wässrige Lösung wird dann durch Filtration vom Katalysator befreit und unter Vakuum schonend eingeengt. Es verbleibt ein Roh-endo-Nortropin, das, wenn notwendig, aus Aceton umkristallisiert wird.

Die Vorteile des erfindungsgemäßen Verfahrens gegenüber dem Stand der Technik lassen sich wie folgt darstellen:

Das Verfahren zum Herstellen von endo-Nortropin umgeht das umständliche Verfahren der Dealkylierung bei dem, ganz gleich nach welcher Methode gearbeitet wird, zum Teil teure, zum Teil umweltschädliche Chemikalien eingesetzt werden.

Es wird durchgehend in wässrigen Lösungen gearbeitet und die Hilfsstoffe wie Katalysatoren und Ionenaustauscher sind leicht abtrennbar und regenerierfähig.

Bei der katalytischen Übertragung von Wasserstoff wird in beiden Schritten bei Raumtemperatur und ohne Druck hydriert.

Dies ist besonders überraschend, da alle bisher bekannten Verfahren mit Überdruck arbeiten.

Das Verfahren arbeitet stereoselektiv, d.h. das Zielprodukt entsteht praktisch vollständig in der gewünschten endo-Form.

Keines der bisher bekannten Verfahren geht beim Herstellen von 3-Nortropanon vom 8-Benzyl-nortropan-3-on-perchlorat aus.

Durch die Salzbildung zum Perchlorat verhalten sich die Tropankörper während der Bearbeitung äußerst stabil.

Weitere Vorteile und Merkmale gehen aus den Unteransprüchen hervor.

Im folgenden werden bevorzugte Ausführungsbeispiele zur Herstellung von 8-Benzyl-nortropan-3-on-perchlorat und des endo-Nortropins anhand der nachfolgenden Formelfließbilder des erfindungsgemäßen Verfahrens beschrieben.

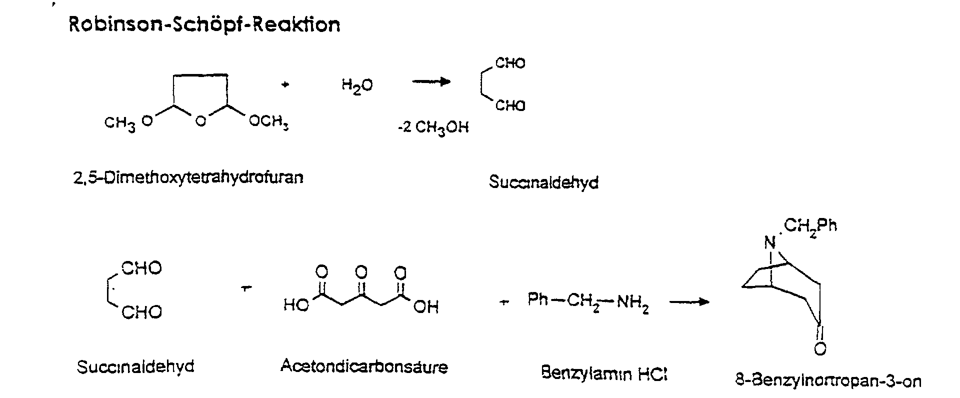

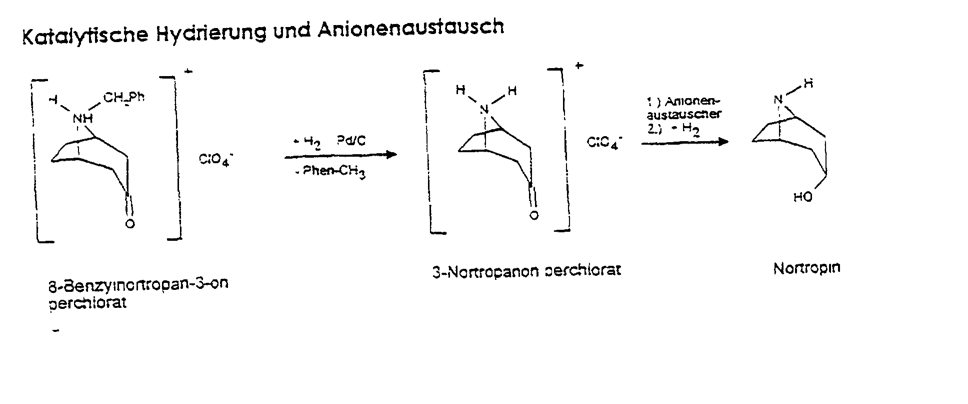

### Beispiel 1

### Verfahren zur Herstellung von 8-Benzyl-nortropan-3-on-perchlorat

51 g entsprechend 0,386 mol 2,5-Dimethoxytetrahydrofuran werden unter Rühren mit 100 ml Wasser gemischt und auf Zusatz von zwei Portionen á 2 ml Salzsäure, 25prozentig, bei Raumtemperatur zum Succinaldehyd hydrolysiert. Nach dem Verdünnen mit Wasser auf ein Volumen von 400 ml gibt man zur gerührten Lösung 58g Acetondicarbonsäure. Nach 5 Minuten Rühren entsteht eine klare gelbe Lösung. Diese versetzt man jetzt portionsweise mit 54,2g Benzylammoniumchlorid, das sich unter weiterem Rühren rasch auflöst. Nun wird die ständig gerührte Lösung mit 12 g Dinatriumhydrogenphosphat auf pH 2 abgepuffert und über Nacht weiter gerührt.

Man klärt die nun trübe Lösung durch Zusatz von 4 g Aktivkohle und Filtration. Durch Zugabe von 33 ml Perchlorsäure (60prozentig) zum Filtrat fällt man das gewünschte Produkt aus.

Es wird abgesaugt, mit wenig Wasser säurefrei gewaschen und an der Luft getrocknet.

Ausbeute 87 g Rohprodukt, Gehalt HPLC 92,5 Prozent, Schmelzpunkt 193 bis 194°C.

Sowohl die entsprechende Elementaranalyse als auch das IR-Spektrum bestätigten das neu aufgefundene Perchloratsalz.

### Beispiel 2

### Hydrierung von 8-Benzyl-nortropan-3-on-perchlorat zum endo-Nortropin

17,5g 8-Benzyl-nortropan-3-on-perchlorat (ca. 90%ig) werden in 180 ml Wasser suspendiert.

Dann setzt man 1,8 g Palladium/Aktivkohle Katalysator 10%ig dazu und hydriert das Ganze bei Raumtemperatur unter Normaldruck mit Wasserstoff. Nach vier Stunden ist die Wasserstoffaufnahme beendet. Man filtriert den Katalysator ab und gibt das klare farblose Filtrat über eine Säule, die einen stark basischen Anionenaustauscher vom Typ 1 gelförmig mit dem Gegenion OH⁻ enthält. Bei einer Austauscherkapazität von ca. 1,4 mmol/ml benötigt man davon etwa 40 ml. Mit drei Portionen ä 20 ml Wasser wäscht man nach, versetzt die aufgefangenen Eluate mit 3 g Raney-Nickel-Katalysator und hydriert das Ganze unter heftigem Turbulieren mit Wasserstoff. Ist die Wasserstoffaufnahme beendet, trennt man den Katalysator ab und engt das klare Filtrat unter Vakuum ein.

Als Rückstand verbleiben 5,6 g rohes endo-Nortropin mit einem Schmelzpunkt von 155 bis 160°C unter Zersetzung.

Versuche haben ergeben, daß die Ausbeuten generell im Bereich von 85 - 100 % der Theorie liegen.

## Patentansprüche

1. Verfahren zur Herstellung von endo-Nortropin , gekennzeichnet durch zweistufige Behandlung von 8-Benzylnortropan-3-on-perchlorat mit katalytisch erregtem Wasserstoff, wobei man das Startprodukt zunächst in wässriger Suspension bei atmosphärischem Druck und Raumtemperatur mit einem Palladium-Katalysator vorhydriert, am Ende der Reaktion den Katalysator durch Filtration zurückgewinnt, das Filtrat über einen Anionenaustauscher schickt und die nunmehr alkalisch reagierende Lösung mit durch Raney-Nickel erregtem Wasserstoff bei atmosphärischem Druck und Raumtemperatur bei 1000 - 1500 Umdrehungen pro Minute turbuliert.

2. Verfahren zur Herstellung von 8-Benzyl-nortropan-3-on-perchlorat als Zwischenprodukt zur Herstellung von endo-Nortropin , gekennzeichnet durch folgende Stufen:
a. Herstellen von 8-Benzyl-nortropan-3-on-perchlorat gemäß an sich bekannter Robinson-Schöpf-Methode aus Dimethoxytetrahydrofuran, Benzylamin und Acetondicarbonsäure in wässriger Lösung;
b. Zugabe einer äquimolaren Menge an Perchlorsäure bei Raumtemperatur unter Ausfällung des Endproduktes.

3. 8-Benzyl-nortropan-3-on-perchlorat , hergestellt gemäß Anspruch 2.

4. Verfahren nach Anspruch 1, dadurch gekennzeichnet, daß das endo-Nortropin aus Aceton umkristallisiert wird.

5. Verfahren nach Anspruch 1, dadurch gekennzeichnet, daß der Palladium-Katalysator, 10% auf Aktivkohle eingesetzt wird.

## Claims

1. Process for the preparation of endo-nortropine, characterized by a two-stage treatment of 8-benzyl-nortropan-3-one perchlorate with catalytically activated hydrogen, the starting product initially being prehydrogenated in aqueous suspension at atmospheric pressure and ambient temperature with a palladium catalyst, at the end of the reaction the catalyst is recovered by filtration, the filtrate is passed over an anion exchanger and the now alkaline reacting solution is rendered turbulent by hydrogen activated by Raney nickel at atmospheric pressure and ambient pressure and 1000 to 1500 revolutions per minute.

2. Process for the preparation of 8-benzyl-nortropan-3-one perchlorate as an intermediate for the preparation of endo-nortropine, characterized by the following stages:
a. preparation of 8-benzyl-nortropan-3-one perchlorate according to the per se known Robinson-Schöpf method from dimethoxytetrahydrofuran, benzyl amine and acetone dicarboxylic acid in aqueous solution,
b. addition of an equimolar quantity of perchloric acid at ambient temperature and accompanied by the precipitation of the end product.

3. 8-benzyl-nortropan-3-one perchlorate, prepared in accordance with claim 2,

4. Process according to claim 1, characterized in that the endo-nortropine is recrystallized from acetone.

5. Process according to claim 1, characterized in that the 10% palladium catalyst is used on activated carbon.

## Revendications

1. Procédé pour la fabrication d'endonortropine, caractérisé par un traitement en deux étapes de perchlorate de 8-benzylnortropan-3-one avec de l'hydrogène activé catalytiquement, dans lequel, en premier lieu, on préhydrogène le produit de départ avec un catalyseur au palladium dans une suspension aqueuse à la pression atmosphérique et à la température ambiante, on récupère le catalyseur par filtration à la fin de la réaction, on fait passer le filtrat dans un échangeur d'anions et on agite à 1000 - 1500 tours par minute la solution, qui présente désormais une réaction alcaline, avec de l'hydrogène activé par du nickel de Raney à la pression atmosphérique et à la température ambiante.

2. Procédé pour la fabrication de perchlorate de 8-benzylnortropan-3-one en tant que produit intermédiaire de l'endonortropine, caractérisé par les étapes suivantes :
a. Fabrication de perchlorate de 8-benzylnortropan-3-one selon la méthode de Robinson-Schöpf, connue en soi, à partir de diméthoxytétrahydrofurane, de benzylamine et d'acide acétone dicarboxylique en solution aqueuse ;
b. Ajout d'une quantité équimolaire d'acide perchlorique à la température ambiante avec précipitation du produit final.

3. Perchlorate de 8-benzylnortropan-3-one fabriqué selon le procédé de la revendication 2.

4. Procédé selon la revendication 1, caractérisé en ce que l'endonortropine est recristallisée à partir d'acétone.

5. Procédé selon la revendication 1, caractérisé en ce que le catalyseur au palladium est utilisé à 10% sur du charbon actif.
